# EUROPEAN PATENT SPECIFICATION

(11) **EP 3 772 378 B1**
(45) Date of publication and mention of the grant of the patent: **13.09.2023**
(21) Application number: 20000223.6
(22) Date of filing: 24.06.2020
(51) Int. Cl.: B02C 18/10, B02C 18/30, B02C 18/36, C12M 1/33

(54) **DISRUPTING AND DISSOCIATING DEVICE OF SOLID BIOLOGIC MATERIALS FOR A REGENERATIVE MEDICAL AND SURGICAL USE**
UNTERBRECHUNGS- UND DISSOZIATIONSVORRICHTUNG VON FESTEN BIOLOGISCHEN MATERIALIEN FÜR EINE REGENERATIVE MEDIZINISCHE UND CHIRURGISCHE VERWENDUNG
DISPOSITIF DE RUPTURE ET DE DISSOCIATION DE MATÉRIAUX BIOLOGIQUES SOLIDES POUR UNE UTILISATION MÉDICALE ET CHIRURGICALE RÉGÉNÉRATIVE

(30) Priority: 06.08.2019 IT 201900014106
(43) Date of publication of application: 10.02.2021
(73) Proprietor: C.T.S.V. S.R.L., 10090 Bruino (TO) (IT); Roggero, Armando, 10090 Reano (TO) (IT)
(72) Inventor: Roggero, Armando, I-10090 Reano (TO) (IT)
(74) Representative: Garavelli, Paolo

(56) References cited:
- EP-A1- 0 623 317
- EP-A1- 0 720 513
- EP-A2- 3 233 289
- WO-A2-2007/089658
- US-A1- 2008 253 223
- US-A1- 2008 285 378
- Samyang Corporation Astm ET AL: "Samyang Corporation -Polycarbonate General Material Status Commercial: Active Availability Asia Pacific Europe North America Features Food Contact Acceptable Good Dimensional Stability Good Electrical Properties Good Flow Good Weather Resistance Low Moisture Absorption Low Temperature Impact Resista", Ultrapolymers, pages 1-2, XP093044555, Retrieved from the Internet: URL:http://catalog.ides.com/Datasheet.aspx ?I=26119&U=0&E=138378 [retrieved on 2023-05-04]

## Description

The present invention refers to a disrupting and dissociating device of solid biologic materials, such as animal, plant tissues and the like, suitable to be used in the regenerative medical and surgical practice.

In particolar, the invention refers to a disrupting and dissociating device for solid biologic tissues, configured in particular to extract cell aggregates lacking metallic residuals and rich of small-sized cells, therefore potentially young, to be used in surgery for healing cronic and/or acute wounds.

WO-A2-2016097960 discloses a known disrupting device for biologic material, comprising a hollow external body defining an internal chamber, a fixed disruption grid having a plurality of microholes equipped with cutting edges transversally housed in the internal chamber in order to define an upper loading chamber superior adapted to be loaded with solid biologic material to be disrupted, a lower collecting chamber adapted to collect the biologic material after having been disrupted, a rotor with blade rotating in the internal chamber, adapted to cooperate with the disruption grid in order to supply and take the biologic material from the upper loading chamber in contact with the disruption grid and therefore create its dissociation through the microholes of the disruption grid.

The microholes of the disruption grid are obtained through a process for punching a metallic plate and have an edge with different cutters; said grid of the disrupting device has the risk of freeing metallic residuals in the disrupted suspension.

US-A1-2008/253223, US-A1-2008/285378, WO-A2-2007/089658, EP-A1-0 720 513 and EP-A1-0 623 317 disclose prior art devices or cutting solutions.

Object of the present invention is improving the prior art devices, by providing a disrupting and dissociating device of biologic materials which allows obtaining cell suspensions and clusters of aggregate cells lacking metallic residuals and therefore usable in numerous medical and surgical applications, said device efficiently performing the disruption of solid biologic substances, by making a delicate cut, which does not damage the cell membranes, also of aggregate cells.

The above and other objects and advantages of the invention, as will appear from the following description, are obtained with a disrupting and dissociating device of solid biologic tissues as claimed in the independent claim. Preferred embodiments and non-trivial variations of the present invention are the subject matter of the dependent claims.

It is intended that all enclosed claims are an integral part of the present description.

It will be immediately obvious that numerous variations and modifications (for example related to shape, sizes, arrangements and parts with equivalent functionality) can be made to what is described, without departing from the scope of the invention, as included in the enclosed claims.

The present invention will be better described by a preferred embodiment thereof, provided as a non-limiting example, with reference to the enclosed drawings, in which:

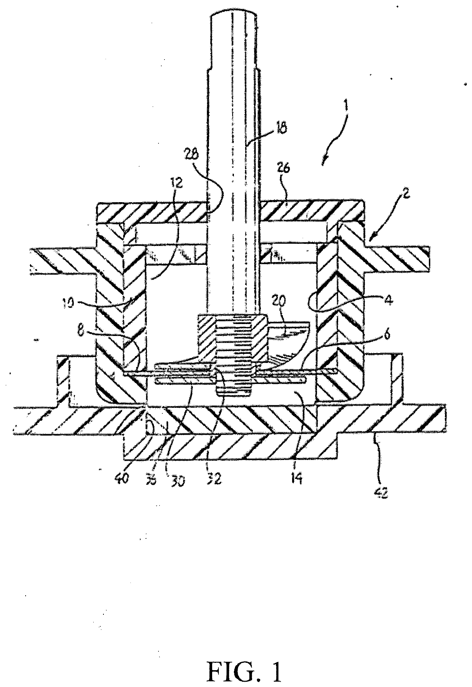

- Figure 1 shows a schematic axial section of a dissociating device according to the invention;

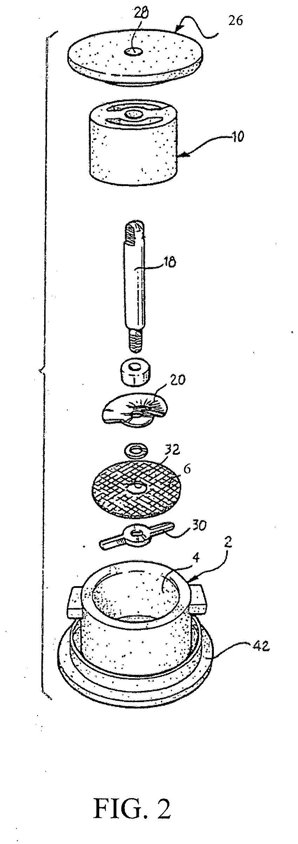

- Figure 2 shows an exploded perspective view of the device of Figure 1;

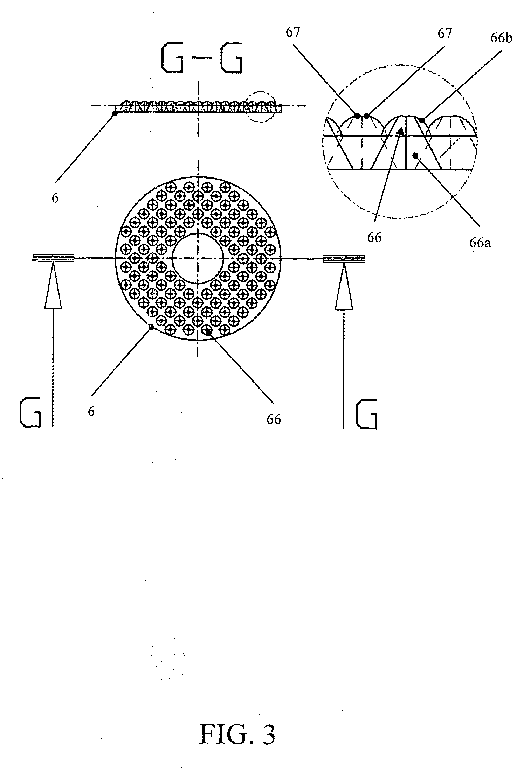

- Figure 3 shows a top view and a sectional view of a part of the device of Figure 1;
- Figure 4 shows a schematic axial view of a dissociating device of the invention in a second embodiment; and
- Figure 5 shows an exploded sectional view and an exploded perspective view of the dissociating device of Figure 4.

With reference to the Figure, the disrupting and dissociating device 1 of biologic materials according to the present invention comprises:
- a case 2 defining at least one internal chamber 4;
- at least one cutting element 6, a flexible holed polymeric disk 6 cutting on the edges of the holes, made through molding of a plastic material, namely TRIREX^{®} 3020IR, said cutting element 6 being housed inside said chamber 4, preferably in a transverse position with respect to the walls of the chamber, in order to define at least one loading chamber 12 of biologic substances to be disrupted and at least one collecting chamber 14 of disrupted biological substances, in particular biologic substances in suspension in a liquid, dissociated from the disrupted solid tissue;
- at least one vane 20 configured to take the biologic substances in contact with the cutting element 6 and, cooperating with the cutting element 6, allow the disruption and the passage from the loading chamber 12 to the collecting chamber 14 of the disrupted biological substances, being at least one between the cutting element 6 and the mobile vane 20, for example rotating, with respect to the other.

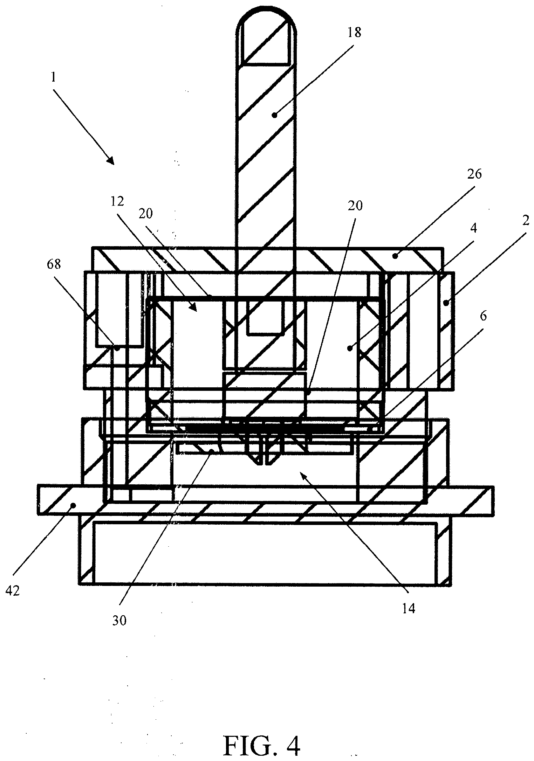

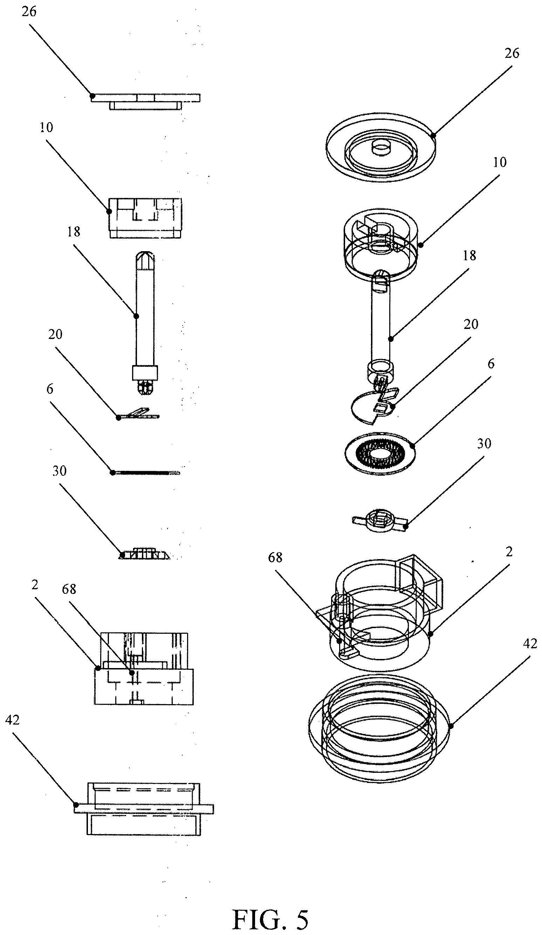

In a preferred embodiment, shown in Figures 4 and 5, the disrupting and dissociating device 1 of the invention comprises at least one capillary channel 68 with connection of the Luer syringe type, connected to the collecting chamber 14 and configured for respectively entering and withdrawing the liquid for the dissociation and the liquid with biologic material in suspension.

The holed disk 6 made through molding of the polymeric material comprises micro-openings 66, having a size included between 20 and 100 um and whose edges compose micro-blades 67 for disruption and dissociation of the solid biologic material.

Said micro-openings 66 are holes having an internal surface 66a shaped as a frustum of cone, preferably a squared base, and an external surface 66b shaped as a dome, each micro-opening 66 of the holed disk 6 being surrounded by four micro-blades 67.

In a preferred embodiment of the disrupting and dissociating device 1 of solid biologic materials of the invention, said device 1 comprises a rotary shaft 18 to which the vane 20 is fastened; said vane 20 is placed in the loading chamber 12 of the chamber 4 and, when rotating, it cooperates with the cutting element 6, for example the holed disk 6 fastened to the case 2, for the disruption of solid biologic material.

Preferably, the cutting element 6, for example the holed polymeric disk 6, is rested along its perimeter edge against a shoulder 8 of the case 2 and is fastened to this latter one through a bush 10 couppled with the internal wall of the case 2. In a preferred way, the upper part of the case 2 is closed by a cover 26 comprising a central hole 28 for passing the shaft 18.

Preferably, the dissociating device 1 further comprises a rotatale propeller 30, for example composed of two or more plane blades, fastened to an end of the shaft 18, which projects in the collecting section 14 through a hole 32 obtained in the cutting element 6, said propeller 30 being configured to create an ascending flow of liquid which cleans the cutting element 6. In a preferred way, the propeller 30 is adjacent and parallel to the lower surface of the cutting element 6.

In a prefered way, the shaft 18 is connected with its upper end to a motor of a known type for its actuation in rotation.

In a preferred way, the case 2 comprises a portion 36 connected to the bottom of the case 2 and configured to be coupled with a seat 40 obtained in a cup-shaped body 42 configured to collect the dissociated material, so that the case 2 is fastened to the cup-shaped body 42.

In the embodiment of the disrupting and dissociating device 1 of the invention shown in Figure 4, at the end of the dissociation of the material, the collection of the material in liquid suspension is performed by sucking liquid through the capillary channel 68 with a connection of the Luer syringe type.

In a preferred way, the holed disk 6 comprising the flexible micro-blades 67 is made of plastic material capable of being sterilized, namely TRIREX^{®} 3020IR, suitable for uses in the medical field.

Advantageously, the disrupting device 1 of solid biologic materials according to the invention guarantees, with the holed disk comprising the micro-blades 67 around every micro-opening 66 in polymer, a cutting property similar to steel but with the addition of the feature of keeping a very delicate cutting, not ruining cell membranes also with aggregate cells and completely avoiding the risk of release of metallic residuals.

## Claims

1. Disrupting and dissociating device (1) of solid biologic materials comprising:
- a case (2) defining at least one internal chamber (4);
- at least one cutting element (6) housed inside said chamber (4) in order to define a loading chamber (12) of biologic substances to be disrupted and a collecting chamber (14) of disrupted biological substances;
- at least one vane (20) configured to take the biologic substances in contact with the cutting element (6) and, cooperating with the cutting element (6), allow the disruption and the passage from the loading chamber (12) to the collecting chamber (14) of the disrupted biological substances, being at least one between the cutting element (6) and the mobile vane (20) with respect to the other;
wherein the cutting element (6) is a holed disk (6) comprising micro-openings (66) whose edges compose micro-blades (67) for disruption and dissociation of the solid biologic material;
**characterized in that** :
- the cutting element (6) is made through molding of polymeric material, namely TRIREX^{®} 3020 IR;
- the micro-openings (66) are holes having an internal surface (66a) shaped as a frustum of cone and an external surface (66b) shaped as a dome;
- the micro-openings (66) of the polymeric disk have a size included between 20 and 100 µm; and
- each micro-opening (66) of the holed polymeric disk (6) is surrounded by four micro-blades (67).

2. Disrupting and dissociating device (1) of solid biologic materials according to claim 1, **characterized in that** it comprises a rotary shaft (18) to which the vane (20) placed in the loading chamber (12) of the chamber (4) is fastened, and rotating with respect to the cutting element (6).

3. Disrupting and dissociating device (1) of solid biologic materials according to any one of the previous claims, **characterized in that** the cutting element (6) is housed inside the chamber (4) in a transverse position with respect to the walls of the chamber (4).

4. Disrupting and dissociating device (1) of solid biologic materials according to any one of the previous claims, **characterized in that** the case (2) comprises a portion (36) connected to the bottom of the case (2) and configured to be coupled with a seat (40) obtained in a cup-shaped body (42) so that the case (2) is fastened to the cup-shaped body (42), configured to collect the disrupted material in liquid suspension.

5. Disrupting and dissociating device (1) of solid biologic materials according to any one of the previous claims, **characterized in that** it comprises a capillary channel (68) connected to the collecting chamber (14) and configured to enter and withdraw liquid.

## Patentansprüche

1. Desintegrator- und Dissoziatorvorrichtung (1) für feste biologische Materialien, umfassend:
- ein Gehäuse (2), das mindestens eine Innenkammer (4) begrenzt;
- mindestens ein Schneidelement (6), das in der Kammer (4) untergebracht ist, um eine Ladekammer (12) für die aufzubrechenden biologischen Substanzen und eine Sammelkammer (14) für die aufgebrochenen biologischen Substanzen zu definieren;
- mindestens eine Klinge (20), die so konfiguriert ist, dass sie die biologischen Substanzen mit dem Schneidelement (6) in Kontakt bringt und im Zusammenwirken mit dem Schneidelement (6) den Zerfall und den Durchgang von der Ladekammer (12) zur Sammelkammer ermöglicht (14) der zerkleinerten biologischen Substanzen, wobei mindestens eines von dem Schneidelement (6) und der Klinge (20) relativ zueinander beweglich ist;
wobei das Schneidelement (6) eine perforierte Scheibe (6) ist, die Mikroöffnungen (66) umfasst, deren Kanten Mikroklingen (67) zum Zerfall und zur Dissoziation des festen biologischen Materials bilden;
**dadurch gekennzeichnet, dass**:
- das Schneidelement (6) wird durch Formen eines Polymermaterials hergestellt, d. h. TRIREX^{®} 3020 IR;
- die Mikroöffnungen (66) sind Löcher mit einer Innenfläche (66a) in Form eines Kegelstumpfes und einer Außenfläche (66b) in Form einer Kuppel;
- die Mikroöffnungen (66) der Polymerscheibe haben eine Größe zwischen 20 und 100 µm; und
- jede Mikroöffnung (66) der perforierten Polymerscheibe (6) von vier Mikrolamellen (67) umgeben ist.

2. Desintegrator- und Dissoziatorvorrichtung (1) für feste biologische Materialien nach Anspruch 1, **dadurch gekennzeichnet, dass** sie eine rotierende Welle (18) aufweist, an der die in der Ladekammer (12) der Kammer (4) befindliche Klinge (20) befestigt ist. feststehend ist, und sich gegenüber dem Schneidelement (6) dreht.

3. Desintegrator- und Dissoziatorvorrichtung (1) für feste biologische Materialien nach einem der vorhergehenden Ansprüche, **dadurch gekennzeichnet, dass** das Schneidelement (6) im Inneren der Kammer (4) in einer Querposition in Bezug auf die Wände der Kammer untergebracht ist Kammer (4).

4. Desintegrator- und Dissoziatorvorrichtung (1) für feste biologische Materialien nach einem der vorhergehenden Ansprüche, **dadurch gekennzeichnet, dass** das Gehäuse (2) einen Abschnitt (36) aufweist, der mit dem Boden des Gehäuses (2) verbunden und zur Kopplung ausgebildet ist mit einem Sitz (40), der in einem becherförmigen Körper (42) ausgebildet ist, so dass das Gehäuse (2) am becherförmigen Körper (42) befestigt ist und so konfiguriert ist, dass er das dissoziierte Material in flüssiger Suspension auffängt.

5. Desintegrator- und Dissoziatorvorrichtung (1) für feste biologische Materialien nach einem der vorhergehenden Ansprüche, **dadurch gekennzeichnet, dass** sie einen Kapillarkanal (68) aufweist, der mit der Sammelkammer (14) verbunden und für die Injektion und Entnahme von Flüssigkeit ausgelegt ist.

## Revendications

1. Dispositif désintégrateur et dissociateur (1) de matières biologiques solides comprenant:
- un carter (2) définissant au moins une chambre interne (4);
- au moins un élément de coupe (6), logé à l'intérieur de ladite chambre (4) de manière à définir une chambre de chargement (12) des substances biologiques à broyer et une chambre de collecte (14) des substances biologiques broyées;
- au moins une lame (20) configurée pour amener les substances biologiques en contact avec l'élément de coupe (6) et, coopérant avec l'élément de coupe (6), permettre la désagrégation et le passage de la chambre de chargement (12) à la chambre de collecte (14) des substances biologiques désintégrées, au moins l'un parmi l'élément coupant (6) et la lame (20) étant mobile par rapport à l'autre;
dans lequel l'élément coupant (6) est un disque perforé (6) comportant des micro-ouvertures (66) dont les bords constituent des micro-lames (67) de désagrégation et de dissociation du matériel biologique solide;
**caractérisé par le fait que**:
- l'élément de coupe (6) est réalisé par moulage d'un matériau polymère, à savoir le TRIREX^{®} 3020 IR;
- les micro-ouvertures (66) sont des trous ayant une surface interne (66a) en forme de tronc de cône et une surface externe (66b) en forme de dôme;
- les micro-ouvertures (66) du disque polymère ont une taille comprise entre 20 et 100 µm; et
- chaque micro-ouverture (66) du disque polymère perforé (6) est entourée de quatre micro-lames (67).

2. Dispositif désintégrateur et dissociateur (1) de matières biologiques solides selon la revendication 1, **caractérisé en ce qu'**il comprend un arbre rotatif (18) auquel la lame (20) située dans la chambre de chargement (12) de la chambre (4) est fixe, et tournant par rapport à l'élément de coupe (6).

3. Dispositif désintégrateur et dissociateur (1) de matières biologiques solides selon l'une quelconque des revendications précédentes, **caractérisé en ce que** l'élément de coupe (6) est logé à l'intérieur de l'enceinte chambre (4) en position transversale par rapport aux parois de l'enceinte chambre (4).

4. Dispositif désintégrateur et dissociateur (1) de matières biologiques solides selon l'une quelconque des revendications précédentes, **caractérisé en ce que** l'enveloppe (2) comprend une partie (36) reliée au fond de l'enveloppe (2) et configurée pour se coupler avec un siège (40) formé dans un corps en forme de coupelle (42) de sorte que le boîtier (2) est fixé au corps en forme de coupelle (42), configuré pour recueillir le matériau dissocié en suspension liquide.

5. Dispositif désintégrateur et dissociateur (1) de matières biologiques solides selon l'une quelconque des revendications précédentes, **caractérisé en ce qu'**il comporte un canal capillaire (68) relié à la chambre de collecte (14) et configuré pour l'injection et le soutirage de liquide.
